# EUROPÄISCHE PATENTANMELDUNG

(11) **EP 3 095 475 A1**
(43) Veröffentlichungstag der Anmeldung: **23.11.2016**
(21) Anmeldenummer: 15001528.7
(22) Anmeldetag: 21.05.2015
(51) Int. Cl.: A61M 1/16

(54) **DIALYSEKONZENTRATBEHÄLTER**

(71) Anmelder: Ritter GmbH, 86830 Schwabmünchen (DE)
(72) Erfinder: Ritter, Frank, 87745 Eppishausen (DE)
(74) Vertreter: Gallo, Wolfgang

(57) **Zusammenfassung**

Dialysekonzentratbehälter für den Einmalgebrauch, mit einem mit Dialysekonzentrat befüllbaren Behälterteil (1, 2) und einem damit verbundenen Anschlußteil (13, 3) zum Anschließen des Dialysekonzentratbehälters an ein Dialysegerät, wobei der Anschlußteil (13, 3) seitwärts beabstandete Fluidanschlußelemente (31) zur Verbindung mit einem Fluidauslaß und einem Fluideinlaß des Dialysegeräts aufweist wobei der Behälterkörper (1, 2) als steife Box ausgebildet ist, dass weiter der Verbindungsteil (13, 3) am unteren Ende des Behälterkörpers angeordnet ist, und dass der Verbindungsteil (13,3) durch einen Plattenkörper gebildet ist, der in einen Anschlußblock des Dialysegeräts einspannbar ist, und an dessen Unterseite die Fluidanschlußelemente (31) gebildet sind, von denen eines mit einem Flüssigkeitseinlaß und eines mit einem Flüssigkeitsauslaß des Behälterkörpers (1) verbunden ist.

## Beschreibung

Die Erfindung betrifft einen Dialysekonzentratbehälter für den Einmalgebrauch, der mit einer für eine Dialysebehandlung vorgesehenen Menge eines Dialysekonzentrats wie Bicarbonat gefüllt ist und an einen Anschlußblock eines Dialysegeräte mit zwei seitlich beabstandeten Fluidanschlüssen ankuppelbar ist.

Ein solcher Dialysekonzentratbehälter ist aus der EP 1 642 614 B1 bekannt. Der bekannte Behälter besteht aus einem Verbindungsteil, der mit Ein- und Auslaßstutzen versehen ist und zum Verbinden des Behälters mit einem Anschlußblock des Dialysegeräts dient, und der einen Behälterteil umfaßt, der sich unten an den Verbindungteil anschließt und vorzugsweise als flexibler Sack ausgebildet ist und das Dialysekonzentrat aufnimmt.

Dieser bekannte Dialysekonzentratbehälter ist also dafür konzipiert, hängend am Anschlußblock des Dialysegeräts angekuppelt zu werden. Die hängende Anordnung erleichtert zwar das Ankuppeln des Behälters am Dialysegerät, erschwert aber die Entleerung am Ende des Gebrauchs. Die Ausbildung des Behälterteils des bekannten Behälters als flexibler Sack erleichtert zwar das Absaugen von Restflüssigkeit, weil der Behälter kollabieren kann, macht aber die Herstellung aufwendiger und teurer, da der steife Verbindungsteil und der flexible Behälterteil aus zwei verschiedenen Materialien hergestellt und miteinander verbunden werden müssen. Auch der Umstand, dass der Behälterteil erst mit dem Dialysekonzentrat befüllt und dann im befüllten Zustand mit dem darüber angeordneten Verbindungsteil verbunden werden muss, verkompliziert den Herstellungsprozess.

Aufgabe der Erfindung ist es, einen diese Nachteile überwindenden Dialysekonzentratbehälter zu schaffen.

Diese Aufgabe wird gemäß der Erfindung durch den im Anspruch 1 angegebenen Dialysekonzentratbehälter gelöst. Vorteilhafte Ausgestaltungen und Weiterbildungen der Erfindung sind Gegenstand der Unteransprüche.

Der erfindungsgemäße Dialysekonzentratbehälter ist als steifer Behälter aus Kunststoff ausgebildet und nicht für hängende, sondern für stehende Montage am Anschlußblock des Dialysegeräts konzipiert. Seine Bauteile sind sämtlich im Spritzgußverfahren aus demselben Material herstellbar und durch Schweißverfahren wie beispielsweise Ultraschallschweißen auf einfache Weise miteinander verbindbar. Insbesondere ist dadurch auch das Befüllen des Behälters mit dem Dialysekonzentrat und das anschließende Verschließen besonders einfach, denn der steife Behälter kann stehend befüllt werden und bedarf keiner Aufhängung, und der Deckel kann eine einfache Deckelplatte sein, da alle Anschlüsse sich unten am Behälter befinden.

Der erfindungsgemäße Dialysekonzentratbehälter besteht aus einem Behälterkörper mit einer oberen, durch einen damit zu verbindenden Deckel verschließbaren Einfüllöffnung und einem unteren Deckel, der mit dem unteren Endbereich des Behälterkörpers eine Anschlußplatte mit Fluidverbindungsstutzen zum Anschluß an den Montageblock des Dialysegeräts aufweist.

Die Erfindung wird nachstehend mit Bezug auf die anliegenden Zeichnungen in näheren Einzelheiten beschrieben, in denen zeigt:
- Fig. 1: in perspektivischer Ansicht der komplette Dialysekonzentratbehälter nach der Erfindung,
- Fig. 2: ebenfalls in perspektivischer Ansicht den kopfstehenden kompletten Dialysekonzentratbehälter,
- Fig. 3: in perspektivischer Ansicht den Behälterkörper ohne oberen und unteren Deckel,
- Fig. 4: in perspektivischer Ansicht den Behälterkörper kopfstehend ohne oberen und unteren Deckel,

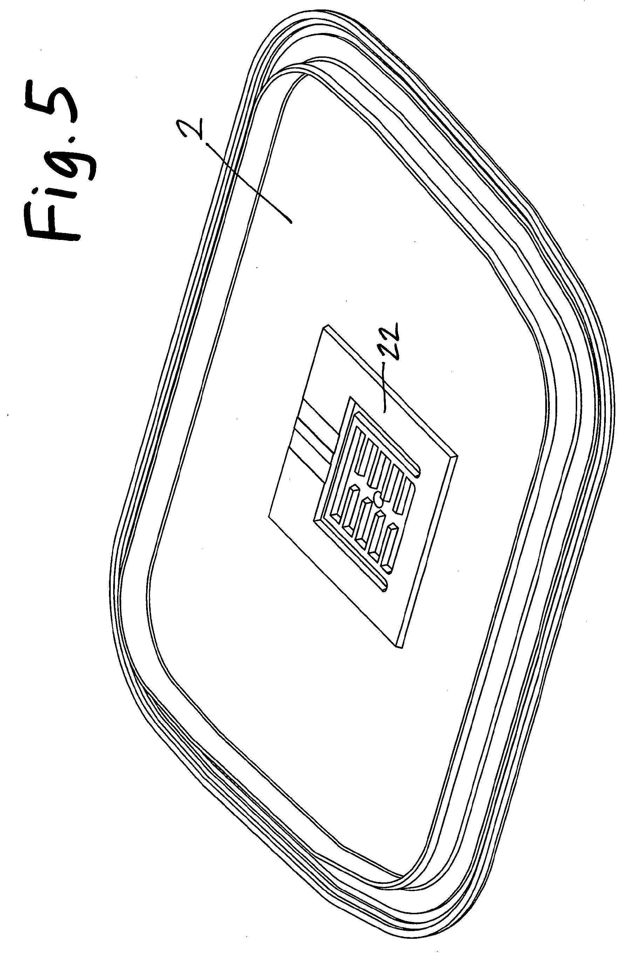

- Fig. 5: den oberen Behälterdeckel in perspektivischer Untersicht,
- Fig. 6: den unteren Behälterdeckel in perspektivischer Ansicht, und
- Fig. 7: den unteren Deckel mit eingesteckten Filterscheiben.

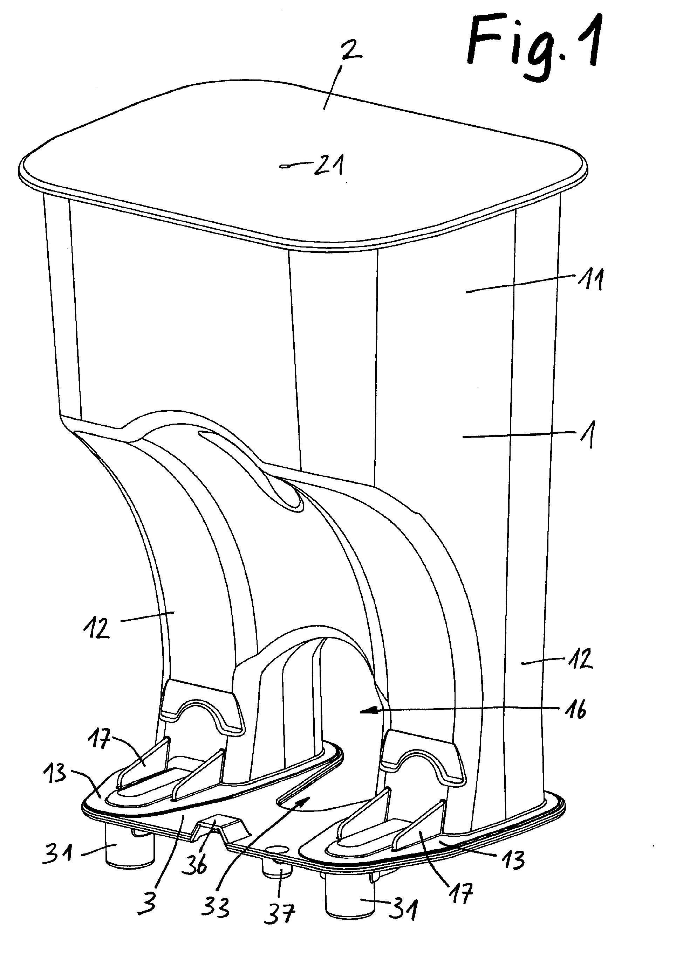

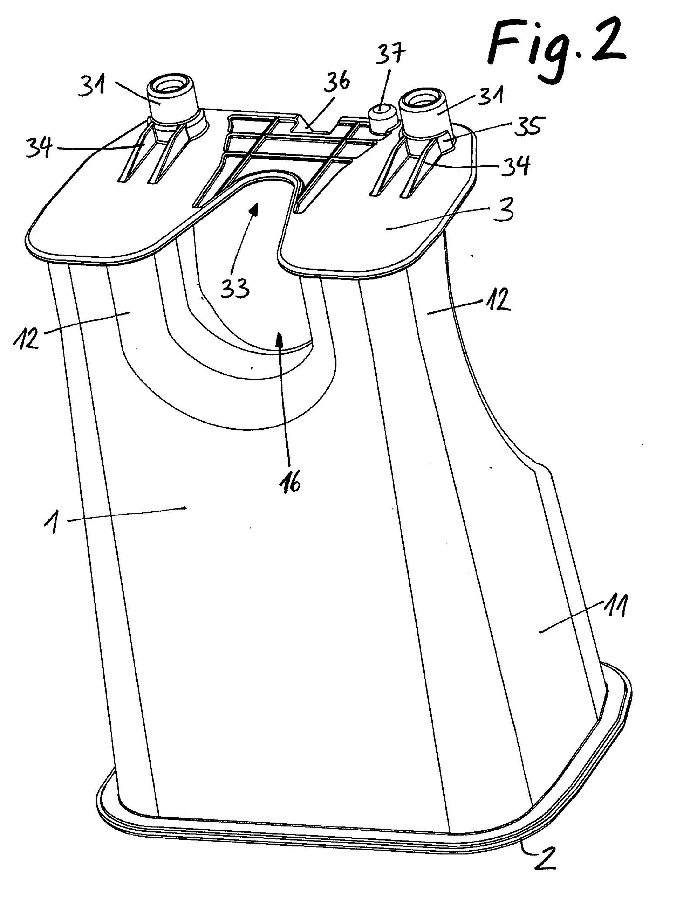

Gemäß den Fig. 1 und 2, die den erfindungsgemäßen Dialysekonzentratbehälter in zwei verschiedenen Ansichten jeweils vollständig zeigen, besteht der Behälter aus einem Behälterkörper 1, einem oberen Deckel 2 und einem unteren Deckel 3.

Die Fig. 1 und 2 zeigen den Dialysekonzentratbehälter jeweils mit Blick auf dessen Rückseite, also dessen im Gebrauch dem Dialysegerät zugewandte Seite.

Der Behälterkörper 1 hat in seinem oberen Bereich 11 einen etwa rechteckigen Querschnitt und geht in seinem unteren Bereich in zwei seitlich beabstandete, als hohle Pfosten ausgebildete Beine 12 über.

Die beiden Beine 12 verlaufen im vorderen Bereich des Behälterkörpers 1, der in den Darstellungen nach den Fig. 1 und 2 mit Blick auf die Rückseite des Dialysekonzentratbehälters hinten liegt. Wie man in den Darstellungen sieht, verjüngt sich der Behälterkörper 1 vom unteren Ende seines oberen Bereichs 11 an von seiner Rückseite aus konkav bogenförmig nach vorn, um in die beiden Beine 12 überzugehen.

Die bogenförmige Verjüngung des unteren Bereichs des Behälterkörpers 1 zu den vorne liegenden Beinen 12 schafft den notwendigen Freiraum für die Andockmechanik des Dialysegeräts, wenn der Dialysekonzentratbehälter an das Dialysegerät angekuppelt wird, so dass der angekuppelte Behälter mit seinem oberen Bereich eng am Dialysegerät anliegen kann.

Der Behälterkörper 1 ist an seinem oberen Ende offen, so dass ein einfaches Einfüllen des Dialysekonzentrats erfolgen kann. Diese obere Behälteröffnung wird dann mit einem Deckel 2 verschlossen, der mit dem oberen Rand des Behälterkörpers 1 ultraschallverschweißt oder auf andere geeignete Weise damit verbunden wird.

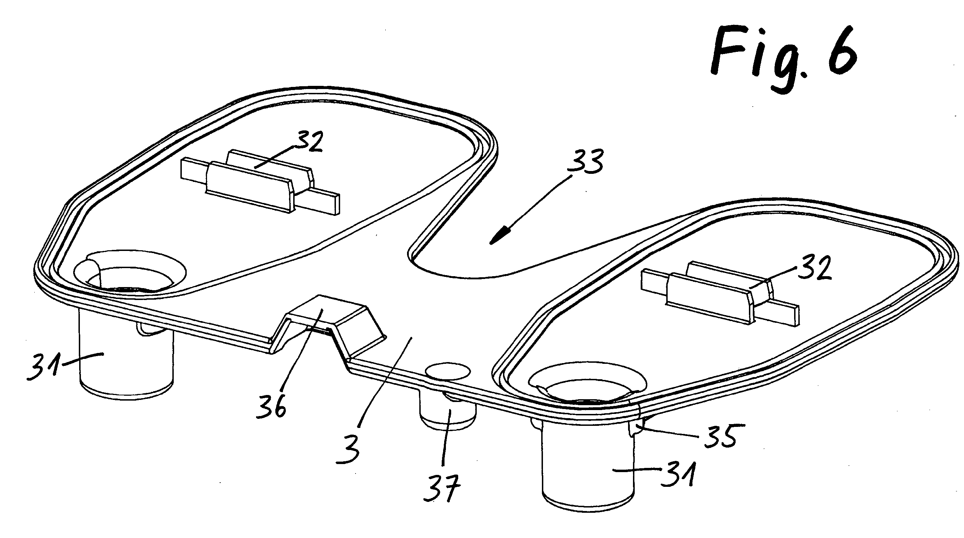

Der obere Deckel 2 hat ein Belüftungsloch 21, wie in Fig. 1 sichtbar ist. An der Unterseite des Deckels 2 befindet sich, wie aus der perspektivischen Untersicht nach Fig. 6 ersichtlich ist, ein Folienventil 22 mit einer das Belüftungsloch 21 verschließenden und bei einem gewissen Unterdruck aufreißenden Folie, die aber der Sichtbarkeit der zugehörigen Struktur am Deckel wegen weggelassen ist. Das Belüftungsloch 21 und das zugehörige Folienventil 22 erleichtern nach Gebrauch des Dialysekonzentratbehälters die Restentleerung, so dass aufgrund des Luftzutritts sämtliche Behandlungsflüssigkeit zuverlässig aus dem Behälter ablaufen kann, ohne dass darin eine Restmenge verbleibt.

Am unteren Ende des Behälterkörpers 1 sind an die Öffnungsränder der hohlpfostenförmigen Beine 12 horizontale Plattenelemente 13 angeformt, die nach rückwärts, also in der Gebrauchslage in Richtung zum Dialysegerät hin vorspringen. Diese bilden zusammen mit dem komplementär ausgebildeten, aber eine durchgehende Platte bildenden unteren Deckel 3, der in Fig. 6 separat dargestellt ist, einen Anschlußteil zum Anschluß des Dialysekonzentratbehälters an das Dialysegerät. Die Plattenelemente 13 könnten auch zu einer durchgehenden Platte verbunden sein.

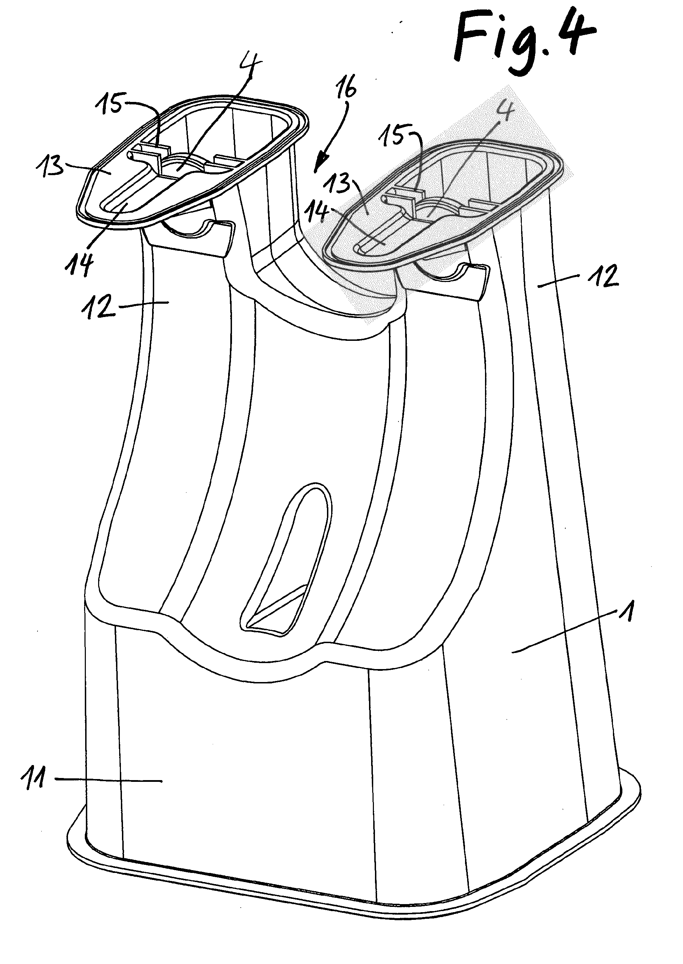

Aus der kopfstehenden Darstellung des Behälterkörpers 1 nach Fig. 4 ist ersichtlich, wie die Plattenelemente 13 beschaffen sind. Man erkennt die offenen Ausmündungen der Beine 12, an die sich jeweils ein in dem jeweiligen Plattenelement 13 eingeformter flacher Kanal 14 anschließt.

Der in Fig. 6 in perspektivischer Draufsicht dargestellte, als durchgehende Platte ausgebildete untere Deckel 3 liegt mit seiner Oberseite an den Unterseiten der Plattenelemente 13 an, so dass er zusammen mit diesen die Kanäle 14 begrenzt. Am unteren Deckel 3 sind zwei seitwärts beabstandete, abwärts ragende Rohrstutzen 31 angeformt, die, wenn der untere Deckel 3 mit den Plattenelementen 13 verbunden ist, mit jeweils einem der Kanäle 14 in Verbindung stehen.

Der untere Deckel 3 kann ebenfalls durch Ultraschallschweißen oder auf andere geeignete Weise mit den Plattenelementen 13 des Behälterkörpers 1 verbunden werden.

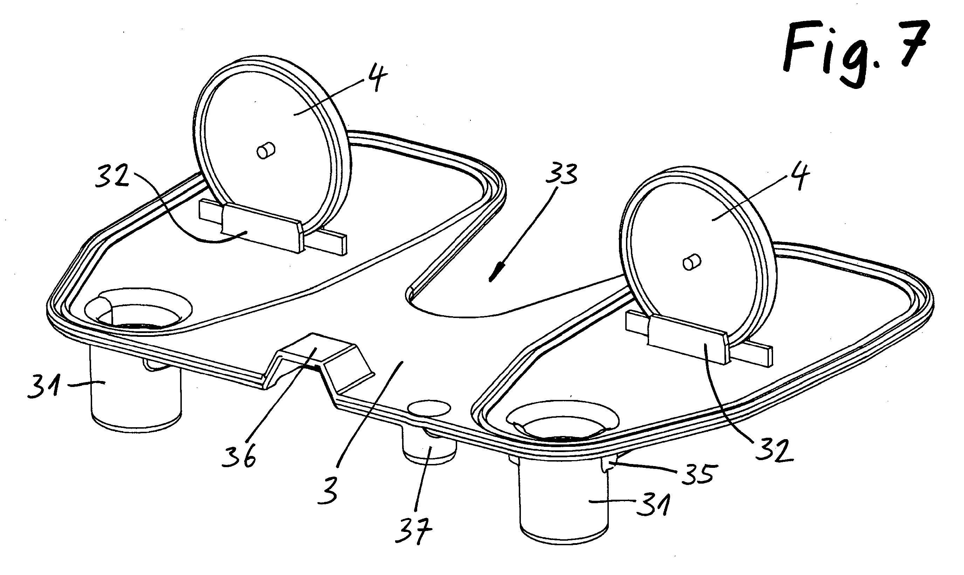

In Fig. 4, welche den Behälterkörper ohne oberen und unteren Deckel kopfstehend zeigt, erkennt man an den in dieser Darstellung oben liegenden Ausmündungen der unteren Enden der Beine 12 des Behälterkörpers 1 in das Innere der hohlen Beine 12 eintauchende Stege 15, die zur Halterung jeweils einer beim Ausführungsbeispiel kreisrunden Filterscheibe 4 dienen. In Fig. 6 sieht man an der Oberseite des Deckels 3 entsprechende Stegformationen 32 die komplementär zu den Stegen 15 in den Beinen 12 des Behälterkörpers ausgebildet sind und ebenfalls der Halterung der Filterscheiben 4 dienen. In Fig. 7 ist der Deckel in gleicher Weise wie in Fig. 6 dargestellt, wobei zur Veranschaulichung die Filterscheiben 4 in die Stegformationen 32 eingesteckt sind. Durch die so gehalterten Filterscheiben 4 ist der Innenraum der Beine 12 des Behälterkörpers 1 von den Kanälen 14 abgetrennt, und die Filterscheiben 4 sind so ausgebildet, dass zwar Flüssigkeit hindurchtreten kann, aber kein festes Dialysekonzentrat.

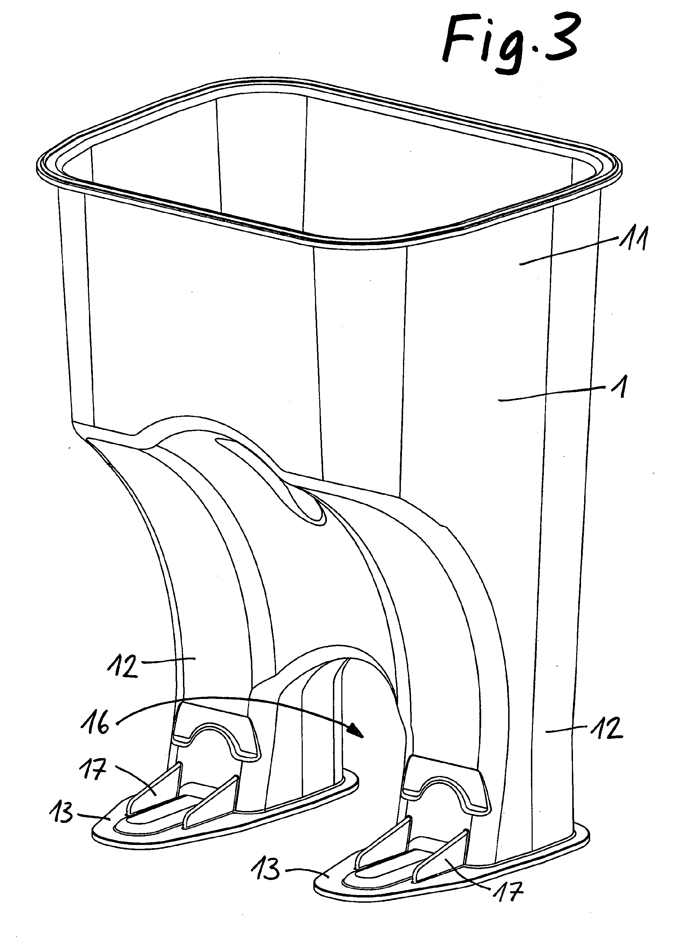

Wie in den Fig. 1 und 3 erkennbar ist, sind Versteifungsrippen 17 am Behälterkörper 1 vorgesehen, welche die Plattenelemente 13 mit den Beinen 12 verbinden. Durch diese Versteifung wird vermieden, dass der Behälter im Betrieb, wenn er mit Behandlungsflüssigkeit gefüllt ist, unter dem dadurch erhöhten Gewicht unter Ausbiegen des Anschlussteils etwas abkippen kann.

Weitere Versteifungsrippen 34 sind, wie in Fig. 2 ersichtlich ist, an der Unterseite des unteren Deckels 3 angeordnet, welche an den Rohrstutzen 31 angeformte Anschlagelemente 35 mit der Deckelunterseitenfläche verbinden. Sie dienen dem gleichen Zweck wie die Versteifungsrippen 17.

Eine dachförmige Formation 36 am unteren Deckel 3 bildet einen an den Anschlussblock des Dialysegeräts angepasster Auflagepunkt, der mit zwei weiteren Stellen an der Unterseite des unteren Deckels 3 als Dreipunkt-Abstützung mit dem Anschlussblock zusammenwirkt.

Ein Zapfen 37 an der Unterseite des unteren Deckels betätigt beim Ankuppeln einen Sensorknopf am Anschlussblock des Dialysegeräts, so dass dieses erkennt, dass ein Dialysekonzentratbehälter angekuppelt ist.

Bei der Fertigung des erfindungsgemäßen Dialysekonzentratbehälters werden der Behälterkörper 1 sowie der obere Deckel 2 und der untere Deckel 3 jeweils separat im Spritzgußverfahren hergestellt. Dann werden in die unteren Öffnungen der Beine 12 des Behälterkörpers 1 die Filterscheiben 4 eingesteckt und dann der untere Deckel 3 aufgesetzt und beispielsweise durch Ultraschallverschweißen mit den Plattenelementen 13 an den unteren Enden der Beine 12 verbunden.

Der so aus dem Behälterkörper 1 und dem damit verbundenen unteren Deckel 3 mit eingesetzten Filterscheiben 4 gebildete Rohbehälter kann dann mit Dialysekonzentrat wie beispielsweise Bicarbonat gefüllt und abschließend durch Anbringen des oberen Deckels 2 verschlossen werden.

Der Dialysekonzentratbehälter kann mit dem aus den Plattenelementen 13 und dem Deckel 3 gebildeten Anschlußteil an ein Dialysegerät angeschlossen werden, wobei die Rohrstutzen 31 in entsprechende Anschlußöffnungen eines Anschlußblocks des Dialysegeräts eintauchen, und wobei der aus den Plattenelementen 13 und dem Deckel 3 gebildete plattenförmige Anschlußteil des Dialysekonzentratbehälters in seinem zwischen den beiden Rohrstutzen 31 liegenden Mittelbereich in einer Klemmvorrichtung des Anschlußblocks des Dialysegeräts eingespannt wird, um die Verbindung des Dialysekonzentratbehälters mit dem Anschlußblock des Dialysegeräts zu sichern.

Der im untersten Bereich des Behälterkörpers 1 zwischen den beiden seitlich beabstandeten Beinen 12 gebildete freie Zwischenraum 16 zusammen mit einem Einschnitt 33 im unteren Deckel 3, der bei mit dem Behälterkörper 1 verbundenem unteren Deckel zwischen den unteren Enden der beiden Beine 12 des Behälterkörpers vom vorne liegenden Rand des unteren Deckels 3 aus nach rückwärts verläuft, bildet eine Durchgriffsöffnung, die es dem Benutzer ermöglicht, ohne Probleme mit den Fingern von der vom Dialysegerät abgewandten Vorderseite hindurchzugreifen, um die Klemmvorrichtung des Anschlußblocks des Dialysegeräts zum Ankuppeln des Dialysekonzentratbehälters an das Dialysegerät oder zum Abnehmen des Dialysekonzentratbehälters vom Dialysegerät zu schließen und zu öffnen.

Im Betrieb dient dann einer der beiden Rohrstutzen 31 als Flüssigkeitseinlaß, und der andere dieser Rohrstutzen dient als Flüssigkeitsauslaß, und die beiden Beine 12 des Behälterkörpers 1 bilden einen Zulauf- und einen Ablaufkanal, so dass der Dialysekonzentratbehälter über die Anschlüsse mit dem Dialysegerät mit Behandlungsflüssigkeit durchströmt und dadurch das granulatförmige Dialysekonzentrat im Behälter gelöst und ausgeschwemmt werden kann.

Am Ende der Behandlung, wenn die Behandlungsflüssigkeit vom Dialysegerät abgezogen wird, bewirkt der entstehende Unterdruck im Behälterinneren das Aufreißen des Folienventils 22 an der Innenseite des oberen Deckels 2 und damit die Belüftung des Behälterinneren, so dass die Behandlungsflüssigkeit vollständig ablaufen kann, was durch die Schwerkraft unterstützt wird, da das gesamte Behälterinnenvolumen sich oberhalb der Fluidanschlüsse des Dialysegeräts befindet.

## Patentansprüche

1. Dialysekonzentratbehälter für den Einmalgebrauch, mit einem mit Dialysekonzentrat befüllbaren Behälterteil (1, 2) und einem damit verbundenen Anschlußteil (13, 3) zum Anschließen des Dialysekonzentratbehälters an ein Dialysegerät, wobei der Anschlußteil (13, 3) seitwärts beabstandete Fluidanschlußelemente (31) zur Verbindung mit einem Fluidauslaß und einem Fluideinlaß des Dialysegeräts aufweist,
**dadurch gekennzeichnet, dass** der Behälterteil (1,2) als steife Box ausgebildet ist, dass weiter der Verbindungsteil (13, 3) am unteren Ende des Behälterteils (1, 2) angeordnet ist, und dass der Verbindungsteil (13,3) durch einen Plattenkörper gebildet ist, der in einen Anschlußblock des Dialysegeräts einspannbar ist, und an dessen Unterseite die Fluidanschlußelemente (31) gebildet sind, von denen eines mit einem Flüssigkeitseinlaß und eines mit einem Flüssigkeitsauslaß des Behälterteils (1, 2) verbunden ist.

2. Dialysekonzentratbehälter nach Anspruch 1, wobei der Behälterteil (1, 2) aus einem oben offenen Behälterkörper (1) und einem damit verbindbaren und diesen oben abschließenden Deckel (2) besteht.

3. Dialysekonzentratbehälter nach Anspruch 1 oder 2, wobei der Behälterteil (1, 2) einen oberen kastenartigen Bereich (11) hat und in seinem unteren Bereich in zwei seitlich beabstandete, mit dem Anschlußteil (13, 3) verbundene und als hohle Pfosten ausgebildete Beine (12) übergeht, von denen eines den Flüssigkeitseinlaß und das andere den Flüssigkeitsauslaß des Behälterkörpers bildet.

4. Dialysekonzentratbehälter nach Anspruch 3, wobei der Behälterkörper (1) an die offenen unteren Enden der Beine (12) angeformte horizontale Plattenelemente (13) aufweist, die zusammen mit einem damit verbindbaren unteren Deckel (3) den Anschlußteil (13, 3) bilden, und wobei die Anschlußelemente (31) an dem unteren Deckel (3) angeformt sind, und wobei in den Plattenelementen (13) und/oder im unteren Deckel (3) Kanäle (14) eingeformt sind, die jeweils eines der Anschlußelemente (31) mit dem unteren Ende eines der beiden hohlen Beine (12) verbinden.

5. Dialysekonzentratbehälter nach Anspruch 3 oder 4, wobei die Beine (12) im vorderen, in der Gebrauchslage vom Dialysegerät abgewandten Bereich des Behälterteils (1, 2) gebildet sind, der Behälterkörper (1) sich unterhalb seines oberen Bereichs (11) in die Beine (12) übergehend nach vorne verjüngt, und der Anschlußteil (13, 3) von den unteren Enden der Beine (12) aus nach rückwärts zum Dialysegerät hin verläuft.

6. Dialysekonzentratbehälter nach Anspruch 5, wobei ein zwischen den beiden seitlich beabstandeten, als hohle Pfosten ausgebildeten Beinen (12) des Behälterkörpers (1) gebildeter freier Zwischenraum (16) und ein im Bereich zwischen den unteren Enden dieser beiden Beine (12) gebildeter Einschnitt (33) im Anschlußteil (13, 3) zusammen eine Durchgriffsöffnung bilden, die es dem Benutzer ermöglicht, eine Klemmvorrichtung des Dialysegeräts zum Einspannen oder Lösen des Anschlußteils des Dialysekonzentratbehälters zu schließen oder zu öffnen.

7. Dialysekonzentratbehälter nach Anspruch 5 oder 6, wobei der Behälterkörper (1) sich unterhalb seines oberen Bereichs (11) in Gestalt eines konkaven Bogens in die Beine (12) übergehend nach vorne verjüngt.

8. Dialysekonzentratbehälter nach einem der Ansprüche 1 bis 7, wobei zwischen den Fluidanschlußelementen (31) und dem Flüssigkeitseinlaß bzw. dem Flüssigkeitsauslaß des Behälterkörpers (1) jeweils eine Filterscheibe (4) eingesetzt ist, die Flüssigkeit hindurchtreten läßt, jedoch festes Dialysekonzentrat zurückhält.

9. Dialysekonzentratbehälter nach einem der Ansprüche 3 bis 7 und Anspruch 8, wobei die Filterscheiben (4) in die unteren Enden der Beine (12) des Behälterkörpers (1) eingesetzt sind.

10. Dialysekonzentratbehälter nach den Ansprüchen 4 und 8, wobei die Filterscheiben (4) zwischen dem jeweiligen Innenraum der hohlen Beine (12) und dem damit verbundenen Kanal (14) angeordnet sind, und wobei die Filterscheiben durch komplementäre Stegformationen (15, 32) in den hohlen Beinen (12) und am unteren Deckel (3) gehaltert werden.

11. Dialysekonzentratbehälter nach einem der Ansprüche 2 bis 10, wobei im oberen Deckel (2) eine an der Deckelinnenseite durch ein Folienventil (22) abgedeckte Belüftungsöffnung (21) vorgesehen ist, wobei das Folienventil (22) so eingestellt ist, dass es bei Entstehen eines gewissen Unterdrucks im Behälterinneren aufreißt und einen Luftzutritt durch die Belüftungsöffnung (21) ermöglicht.

12. Dialysekonzentratbehälter nach einem der Ansprüche 1 bis 11, wobei der aus einem Behälterkörper (1) und einem oberen Deckel (2) zusammengesetzte Behälterteil (1, 2) und der durch einen unteren Deckel (3) gebildete Anschlußteil als Kunststoffspritzgußteile ausgebildet sind.
